(19) Europäisches Patentamt
European Patent Office
Office européen des brevets

(11) EP 2 854 634 B1

(12) EUROPEAN PATENT SPECIFICATION

(45) Date of publication and mention of the grant of the patent:
**27.04.2016 Bulletin 2016/17**

(51) Int Cl.:
*A61B 5/06* *(2006.01)* *A61B 34/20* *(2016.01)*

(21) Application number: **13734880.1**

(86) International application number:
**PCT/US2013/045885**

(22) Date of filing: **14.06.2013**

(87) International publication number:
**WO 2014/028114 (20.02.2014 Gazette 2014/08)**

(54) **CORRECTION OF SHIFT AND DRIFT IN IMPEDANCE-BASED MEDICAL DEVICE NAVIGATION USING MAGNETIC FIELD INFORMATION**

KORREKTUR VON VERSCHIEBUNG UND ABDRIFT BEI DER IMPEDANZBASIERTEN NAVIGATION EINER MEDIZINISCHEN VORRICHTUNG UNTER VERWENDUNG VON MAGNETFELDINFORMATIONEN

CORRECTION DE DÉCALAGE ET DE DÉRIVE DANS UNE NAVIGATION DE DISPOSITIF MÉDICAL BASÉE SUR IMPÉDANCE UTILISANT DES INFORMATIONS DE CHAMP MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2012 US 201213584197**

(43) Date of publication of application:
**08.04.2015 Bulletin 2015/15**

(73) Proprietor: **St. Jude Medical, Atrial Fibrillation Division, Inc.**
**St. Paul, Minnesota 55117-9913 (US)**

(72) Inventors:
• **KOYRAKH, Lev A.**
  **Plymouth, Minnesota 55442 (US)**
• **VYLKOV, Vasily**
  **Blaine, Minnesota 55434 (US)**
• **OLSON, Eric S.**
  **Maplewood, Minnesota 55119 (US)**

(74) Representative: **Kramer Barske Schmidtchen Patentanwälte PartG mbB**
**European Patent Attorneys**
**Landsberger Strasse 300**
**80687 München (DE)**

(56) References cited:
**US-A1- 2007 016 007 US-A1- 2012 172 702**

• **RAJESH KABRA ET AL: "Recent Trends in Imaging for Atrial Fibrillation Ablation", INDIAN PACING AND ELECTROPHYSIOLOGY JOURNAL, 5 May 2010 (2010-05-05), pages 215-227, XP055048854, ISSN: 0972-6292**

**Description**

BACKGROUND OF THE INVENTION

a. Field of the Invention

This invention relates to a system and method for navigating a medical device within a body. In particular, the instant invention relates to a system and method that enable correction of drift and shift in impedance levels in electric field based position and navigation systems.

b. Background Art

A wide variety of medical devices are inserted into the body to diagnose and treat various medical conditions. Catheters, for example, are used to perform a variety of tasks within human bodies and other bodies including the delivery of medicine and fluids, the removal of bodily fluids and the transport of surgical tools and instruments. In the diagnosis and treatment of atrial fibrillation, for example, catheters may be used to deliver electrodes to the heart for electrophysiological mapping of the surface of the heart and to deliver ablative energy to the surface among other tasks. Catheters are typically routed to a region of interest through the body's vascular system. In a conventional approach, an introducer is used to puncture the skin surface and a sheath having an inner diameter greater than the outer diameter of the catheter is threaded through the vasculature to a region of interest. The catheter is then moved longitudinally through the sheath to the region of interest either manually by a clinician or through the use of electromechanical drive systems.

It is desirable to track the position of medical devices such as catheters as they are moved within the body so that, for example, drugs and other forms of treatment are administered at the proper location and medical procedures can be completed more efficiently and safely. One conventional means to track the position of medical devices within the body is fluoroscopic imaging. Fluoroscopy is disadvantageous, however, because it subjects the patient and physician to undesirable levels of electromagnetic radiation. As a result, medical device navigation systems have been developed to track the position of medical devices within the body. These systems typically rely on the generation of electrical or magnetic fields and the detection of induced voltages and currents on position sensors attached to the medical device and/or external to the body. The information derived from these systems is then provided to a physician through, for example, a visual display.

One conventional medical device navigation system is made available under the trademark "ENSITE NAVX" by St. Jude Medical, Inc. The system is based on the principle that when electrical currents are passed through the thorax a voltage drop occurs across internal organs such as the heart and this voltage drop can be measured and used to determine the position of a medical device within the body. The system includes three pairs of patch electrodes that are placed on opposed surfaces of the body (e.g., chest and back, left and right sides of the thorax, and neck and leg) and form generally orthogonal x, y, and z axes as well as a reference electrode that is typically placed near the stomach and provides a reference value and acts as the origin of the coordinate system for the navigation system. Sinusoidal currents are driven through each pair of patch electrodes and voltage measurements for one or more electrodes associated with the medical device are obtained. The measured voltages are proportional to the distance of the device electrodes from the patch electrodes. The measured voltages are compared to the potential at the reference electrode and a position of the device electrodes within the coordinate system of the navigation system is determined.

The above-described system can be used to provide a substantially accurate indication of the position of the medical device within a body. Electric field based navigation systems, however, are subject to various types of interference that can impact the accuracy of position measurements. For example, the level of electrical impedance in the patient body is not necessarily constant. The impedance can slowly drift or even undergo transient shifts due to, for example, a change in medication leading to drift and/or shift in the detected position of the medical device. Various methods have been proposed to mitigate potential drift or shift including bio-impedance scaling, patch center subtraction and the use of a fixed reference catheter with a reference electrode. Bio-impedance scaling and patch center subtraction help to reduce drift and shift, but do not eliminate all cases of drift and shift. The use of a fixed reference catheter requires insertion of an additional catheter into the body thereby increasing procedure time and the risk of complications. Further, the reference catheter may become dislodged during the procedure.

US patent application publication US 2007/0016007 A1 discloses a navigation system for a surgical device using a combination of an electrical and a magnetic position sensor.

There is thus an ongoing a need for a system and method for navigating a medical device within a body that will minimize and/or eliminate one or more of the above-identified deficiencies.

BRIEF SUMMARY OF THE INVENTION

The present disclosure relates to a system and method for navigating a medical device within a body. In particular, the present disclosure relates to a system and method that reduce or eliminate potential errors in position detection due to drift or shifts in patient impedance.

A system for navigating a medical device within a body includes an electronic control unit configured to determine an operating position for an electrical position sensor on the medical device within a first coordinate system. The first coordinate system is defined by an electric field based positioning system. The electronic control unit is further configured to determine an operating position for a magnetic position sensor on the medical device within a second coordinate system. The second coordinate system is defined by a magnetic field based positioning system. The magnetic position sensor is disposed proximate the electrical position sensor. The electronic control unit is further configured to apply a mapping function correlating the operating positions of the electrical position sensor and the magnetic position sensor. The mapping function generates a mapped position for the magnetic position sensor in the first coordinate system responsive to the operating position of the magnetic position sensor in the second coordinate system. The electronic control unit is further configured to determine an adjusted operating position for the electrical position sensor in the first coordinate system responsive to the mapped position of the magnetic position sensor.

A method for navigating a medical device within a body includes determining an operating position for an electrical position sensor on the medical device within a first coordinate system. The first coordinate system is defined by an electric field based positioning system. The method further includes determining an operating position for a magnetic position sensor on the medical device within a second coordinate system. The second coordinate system is defined by a magnetic field based positioning system. The magnetic position sensor is disposed proximate the electrical position sensor. The method further includes applying a mapping function correlating the operating positions of the electrical position sensor and the magnetic position sensor. The mapping function generates a mapped position for the magnetic position sensor in the first coordinate system responsive to the operating position of the magnetic position sensor in the second coordinate system. The method further includes determining an adjusted operating position for the electrical position sensor in the first coordinate system responsive to the mapped position of the magnetic position sensor.

The system and method enable consistent correction or errors in position measurements due to shift or drift in patient impedance levels. Further, the system and method do not require the use of an additional reference catheter and the resulting increases in procedure time and risks.

The foregoing and other aspects, features, details, utilities, and advantages of the present invention will be apparent from reading the following description and claims, and from reviewing the accompanying drawings.

BRIEF DESCRIPTION OF THE DRAWINGS

Figure 1 is diagrammatic view of one embodiment of a system for navigating a medical device within a body in accordance with the present teachings.

Figure 2 is a cross-sectional view of a portion of an exemplary medical device for use in the system of Figure 1.

Figure 3A-C is a flow-chart diagram illustrating one embodiment of a method for navigating a medical device within a body in accordance with the present teachings.

Figure 4 is a graphical representation illustrating the detected position of an electrical position sensor on a medical device over time with and without use of a system and method in accordance with the present teachings.

DETAILED DESCRIPTION OF THE INVENTION

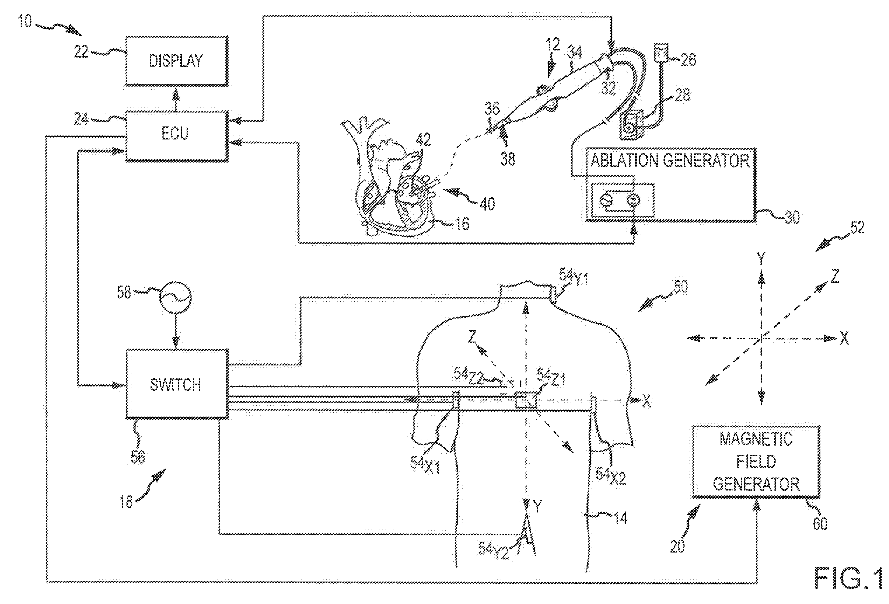

Referring now to the drawings wherein like reference numerals are used to identify identical components in the various views, Fig. 1 illustrates one embodiment of a system 10 for navigating a medical device within a body 12. In the illustrated embodiment, the medical device comprises a catheter 14 and, in particular, an irrigated ablation catheter for use in diagnosis or treatment of cardiac tissue 16 in body 12. It should be understood, however, that a system 10 in accordance with the present teachings may find application in connection with a wide variety of medical devices used within body 12 for diagnosis or treatment. For example, system 10 may be used to navigate an electrophysiological (EP) mapping catheter or an intradcardiac echocardiography (ICE) catheter. Further, it should be understood that the system may be used to navigate medical devices used in the diagnosis or treatment of portions of body 12 other than the tissue 16. System 10 may include an electric field based positioning system 18, a magnetic field based positioning system 20, a display 22 and an electronic control unit (ECU) 24.

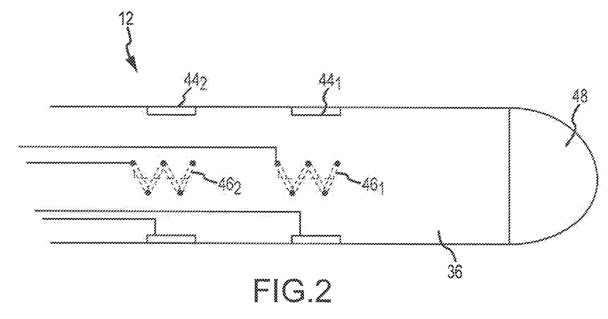

Catheter 14 is provided for examination, diagnosis and treatment of internal body tissues such as cardiac tissue 16. In accordance with one embodiment, catheter 14 comprises an ablation catheter and, more particularly, an irrigated radio-frequency (RF) ablation catheter. It should again be understood, however, that catheter 14 is provided for illustration only and that system 10 could be adapted for use with a variety of catheters including, for example, electrophysiology mapping catheters and intracardiac echocardiograph (ICE) catheters, as well as for use with other types of ablation catheters including those providing different types of ablation energy (e.g., cryoablation, ultrasound, etc.). Catheter 14 is connected to a fluid source 26 having a biocompatible fluid such as saline through a pump 28 (which may comprise, for example, a fixed rate roller pump or variable volume syringe pump with a gravity feed supply from fluid source 26 as shown) for irrigation. Catheter 14 is also electrically connected to an ablation generator 30 for delivery of RF energy. Catheter 14 may include a cable connector or interface 32, a handle 34, a shaft 36 having a proximal end 38 and a distal end 40 (as used herein, "proximal" refers to a direction toward the end of the catheter near the physician, and "distal" refers to a direction away from the physician and (generally) inside the body of a patient) and one or more electrodes 42. Referring to Figure 2, in accordance with one aspect of the present teachings, catheter 14 further includes one or more electrical position sensors $44_1$, $44_2$ and one or more magnetic position sensors $46_1$, $46_2$ for a purposed described hereinbelow. Catheter 14 may also include other conventional components not illustrated herein such as a temperature sensor, additional electrodes, and corresponding conductors or leads.

Connector 32 provides mechanical, fluid and electrical connection(s) for conduits or cables extending from pump 28, and ablation generator 30. Connector 32 is conventional in the art and is disposed at a proximal end 38 of catheter 14.

Handle 34 provides a location for the physician to hold catheter 14 and may further provides means for steering or guiding shaft 36 within body 12. For example, handle 34 may include means to change the length of a guidewire extending through catheter 14 to distal end 40 of shaft 46 to steer distal end 40 and, thus, shaft 36. Handle 34 is also conventional in the art and it will be understood that the construction of handle 34 may vary.

Shaft 36 is an elongated, flexible member configured for movement within body 12. Shaft 36 supports electrodes 42, position sensors $44_1$, $44_2$, $46_1$, $46_2$, associated conductors, and possibly additional electronics used for signal processing or conditioning. Shaft 36 may also permit transport, delivery, and/or removal of fluids (including irrigation fluids and bodily fluids), medicines, and/or surgical tools or instruments. Shaft 36 may be made from conventional materials such as polyurethane and defines one or more lumens configured to house and/or transport electrical conductors, fluids, or surgical tools. Shaft 36 may be introduced into a blood vessel or other structure within body 12 through a conventional introducer sheath. Shaft 36 may then be steered or guided through body 12 to a desired location such as tissue 16 using guide wires or pullwires or other means known in the art including remote control guidance systems.

Electrodes 42 may be provided for a variety of diagnostic and therapeutic purposes including, for example, electrophysiological studies, catheter identification and location, pacing, and cardiac mapping and ablation. Referring to Figure 2, in the illustrated embodiment, catheter 14 includes an ablation tip electrode 48 at distal end 40 of shaft 36. It should be understood, however, that the number, orientation, and purpose of electrodes 42 may vary.

Electrical position sensors $44_1$, $44_2$ are provided for use in determining the position of catheter 14 within body 12. Sensors $44_1$, $44_2$ are conventional in the art. In the illustrated embodiment, sensors $44_1$, $44_2$ comprise electrodes and, in particular, conventional ring electrodes located proximal to the distal end 40 of catheter shaft 36 and tip electrode 48. As sensors $44_1$, $44_2$ move within body 14, and within the electric field generated by system 18, the voltage readings from sensors $44_1$, $44_2$ change thereby indicating the location of sensors $44_1$, $44_2$ within the electric field and with a coordinate system 50 established by system 18. Sensors $44_1$, $44_2$ communicate position signals to ECU 24 through a conventional interface (not shown).

Magnetic position sensors $46_1$, $46_2$ are also provided for use in determining the position of catheter 14 within body 12. Sensors $46_1$, $46_2$ are conventional in the art. In the illustrated embodiment, sensors $46_1$, $46_2$ are coils. As sensors $46_1$, $46_2$ move within body 14, and within the magnetic field generated by system 20, the current output of each sensor $46_1$, $46_2$ changes thereby indicating the location of sensors $46_1$, $46_2$ within the magnetic field and with a coordinate system 52 established by system 20. Sensors $46_1$, $46_2$ may be wound about catheter 14 at or near distal end 40 and may be embedded within the walls of catheter 14 such that sensors $46_1$, $46_2$ are insulated. Alternatively, sensors $46_1$, $46_2$ could be embedded further within catheter 14 as shown in Figure 2, or could be placed at other locations within the catheter 14. Sensors $46_1$, $46_2$ may also have appropriate insulation and/or shielding (e.g., a conductive foil or wire mesh) to cancel potential interferences from other devices near body 12. It should be understood that sensors $46_1$, $46_2$ may take forms other than the form illustrated in Figure 2. Sensors $46_1$, $46_2$ may, for example, comprise any conventional position sensors for detecting changes in magnetic fields including Hall effect sensors, magnetoresistive sensors and sensors made from magnetoresistive materials and piezoelectric materials and the like. Sensors $46_1$, $46_2$ communicate position signals to ECU 24 through a conventional interface (not shown). In accordance with one aspect of the present teachings, each of magnetic position sensors $46_1$, $46_2$ is disposed proximate to a corresponding electrical position sensor $44_1$, $44_2$ such that the detected position of one of sensors 44, 46 may be indicative of the position of the other corresponding sensor 44, 46. The magnetic position sensors $46_1$, $46_2$ may, for example be located from about 1.0 to about 3.0 millimeters from a corresponding electrical position sensor $44_1$, $44_2$ and may be centered between two electrical position sensors $44_1$, $44_2$ which may be spaced about 2.0 to 6.0 millimeters apart.

System 18 is provided to determine the position and orientation of catheter 14 and similar devices within body 12. System 18 may comprise the system made available under the trademark "ENSITE NAVX" by St. Jude Medical, Inc. and described, for example, in U.S. Patent No. 7,263,397 titled "Method and Apparatus for Catheter Navigation and Location Mapping in the Heart." The system is based on the principle that when low amplitude electrical signals are passed through the thorax, body 12 acts as a voltage divider (or potentiometer or rheostat) such that the electrical potential or field strength measured at an electrode such as one of position sensors $44_1$, $44_2$ on catheter 14 may be used to determine the position of the electrode, and therefore catheter 14, relative to a pair of external patch electrodes using Ohm's law and the relative location of a reference electrode (e.g. in the coronary sinus). In one configuration, the system includes three pairs of patch electrodes 54 that are placed on opposed surfaces of body 12 (e.g., chest and back, left and right sides of the thorax, and neck and leg) and form generally orthogonal x, y, and z axes as well as a reference electrode/patch (not shown) that is typically placed near the stomach and provides a reference value and acts as the origin of the coordinate system 50 for the navigation system. Sinusoidal currents are driven through each pair of patch electrodes 54 and voltage measurements for one or more position sensors $44_1$, $44_2$ associated with catheter 14 are obtained. The measured voltages are a function of the distance of the position sensors $44_1$, $44_2$ from the patch electrodes 54. The measured voltages are compared to the potential at the reference electrode and a position of the position sensors $44_1$, $44_2$ within the coordinate system 50 of the navigation system is determined. In accordance with this exemplary system, system 18 may include patch electrodes 54 (namely $54_{X1}$, $54_{X2}$, $54_{Y1}$, $54_{Y2}$, $54_{Z1}$, $54_{Z2}$) a switch 56, and a signal generator 58.

Patch electrodes 54 are provided to generate electrical signals used in determining the position of catheter 14 within three-dimensional coordinate system 50 of system 18. Electrodes 54 may also be used to generate EP data regarding tissue 16. Electrodes 54 are placed orthogonally on the surface of body 12 and are used to create axes specific electric fields within body 12. Electrodes $54_{X1}$, $54_{X2}$ may be placed along a first (x) axis. Similarly, electrodes $54_{Y1}$, $54_{Y2}$ may be placed along a second (y) axis, and electrodes $54_{Z1}$, $54_{Z2}$ may be placed along a third (z) axis. Each of the electrodes 54 may be coupled to multiplex switch 56. ECU 24 is configured through appropriate software to provide control signals to switch 56 and thereby sequentially couple pairs of electrodes 54 to signal generator 58. Excitation of each pair of electrodes 54 generates an electromagnetic field within body 14 and within an area of interest such as the heart. Voltage levels at non-excited electrodes 54 may be filtered and converted and provided to ECU 24 for use as reference values.

System 20 is also provided to determine the position and orientation of catheter 14 and similar devices within body 12. System 20 comprises a system that employs magnetic fields to detect the position of catheter 14 within body 12 such as the system made available under the trademark "GMPS" by MediGuide, Ltd. and generally shown and described in, for example, U.S. Patent No. 7,386,339 titled "Medical Imaging and Navigation System." In such a system, a magnetic field generator 60 may be employed having three orthogonally arranged coils, arranged to create a magnetic field within body 12 and to control the strength, orientation, and frequency of the field. The magnetic field generator 60 may be located above or below the patient (e.g., under a patient table) or in another appropriate location. Magnetic fields are generated by the coils and current or voltage measurements for one or more position sensors $46_1$, $46_2$ associated with catheter 14 are obtained. The measured currents or voltages are proportional to the distance of the sensors $46_1$, $46_2$ from the coils thereby allowing a position of the sensors $46_1$, $46_2$ within a coordinate system 52 of system 20.

Display 22 is provided to convey information to a physician to assist in diagnosis and treatment. Display 22 may comprise one or more conventional computer monitors or other display devices. Display 22 may present a graphical user interface (GUI) to the physician. The GUI may include a variety of information including, for example, an image of the geometry of tissue 16, electrophysiology data associated with the tissue 16, graphs illustrating voltage levels over time for various electrodes 42, and images of catheter 14 and other medical devices and related information indicative of the position of catheter 14 and other devices relative to the tissue 16.

ECU 24 provides a means for controlling the operation of various components of system 10 including catheter 14 and ablation generator 30, switch 56 of system 18, and magnetic generator 60 of system 20. ECU 24 may also provides a means for determining the geometry of tissue 16, electrophysiology characteristics of tissue 16 and the position and orientation of catheter 12 relative to tissue 16 and body 14. ECU 24 also provides a means for generating display signals used to control display 22. ECU 24 may comprise one or more programmable microprocessors or microcontrollers or may comprise one or more application specific integrated circuits (ASICs). ECU 24 may include a central processing unit (CPU) and an input/output (I/O) interface through which ECU 24 may receive a plurality of input signals including signals generated by ablation generator 30, electrodes 42 and position sensors $44_1$, $44_2$, $46_1$, $46_2$ on catheter 14, and patch electrodes 54 of system 18, and generate a plurality of output signals including those used to control and/or provide data to catheter 14, display 22, ablation generator 30, switch 56 of system 18, and generator 60 of system 20.

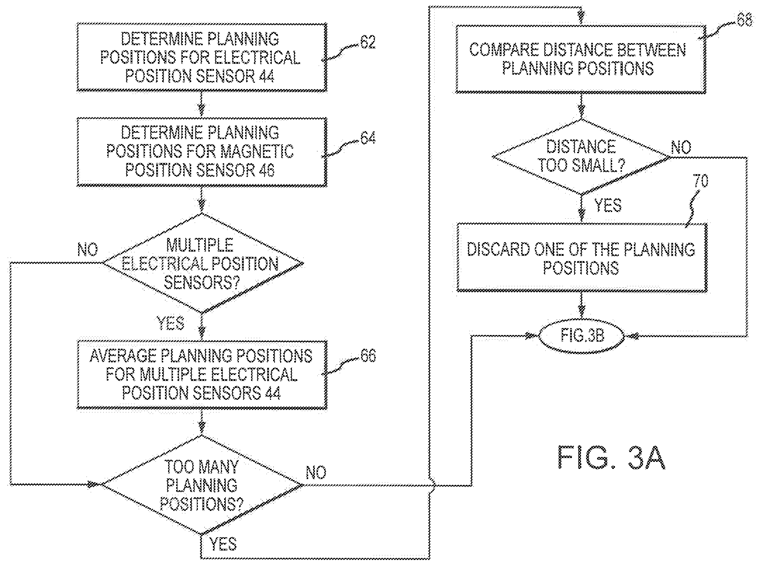

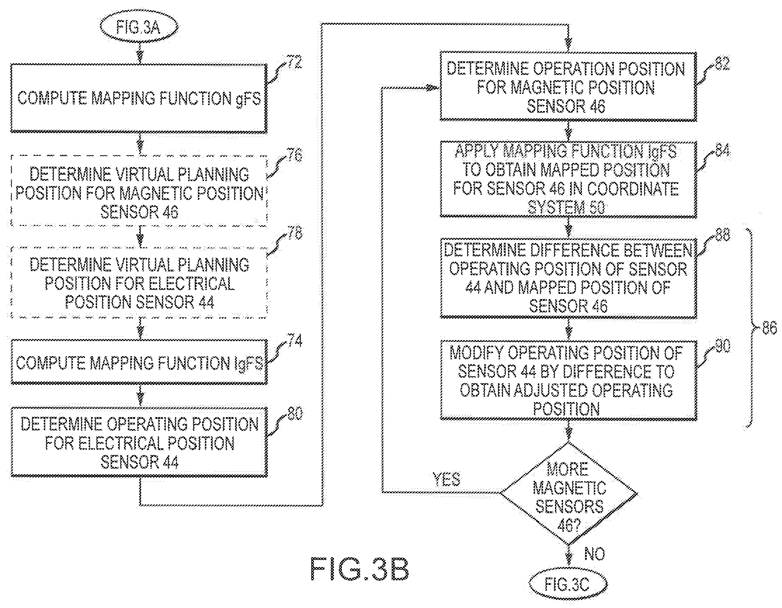

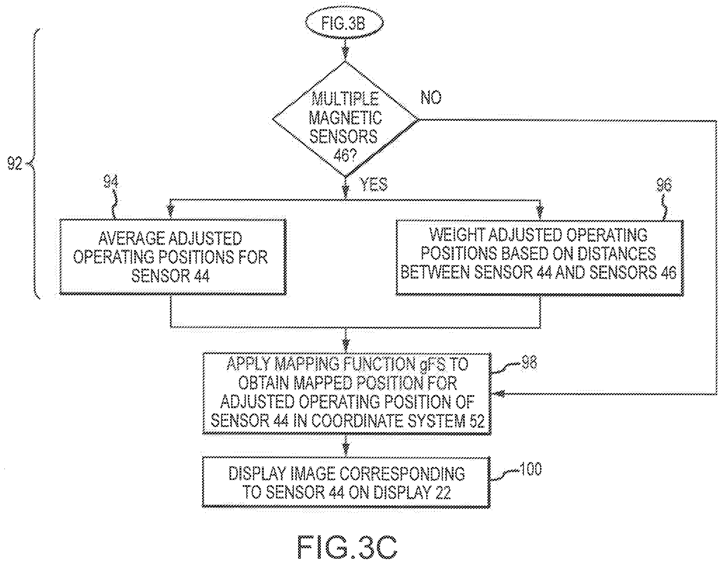

In accordance with the present teachings, ECU 24 may be configured with programming instructions from a computer program (i.e., software) to implement a method for navigating catheter 14 within body 12. The program may be stored in a computer storage medium such as a memory (not shown) that is internal to ECU 24 or external to ECU 24 and may be pre-installed in the memory or obtained from a computer storage medium external to device 10 including from various types of portable media (e.g., compact discs, flash drives, etc.) or file servers or other computing devices accessible through a telecommunications network. Referring to Figure 3, the method may begin with the steps 62, 64 of determining planning positions for an electrical position sensor $44_1$ and a magnetic position sensor $46_1$ within coordinate systems 50, 52, respectively. During a planning stage for a diagnostic or treatment procedure, catheter 14 (or another type of medical device having sensors $44_1$, $46_1$) is disposed within the region of interest (e.g., a chamber of the heart) within coordinate systems 50, 52. Sampling of the sensors $44_1$, $46_1$ is performed to collect pairs of correlated planning positions (also referred to as reference points or fiducials). Each planning position is a pair of three-dimensional electrically or magnetically measured coordinates of the form:

$$\left\{ nX_j, nY_j, nZ_j \right\}$$

and

$$\left\{ gX_j, gY_j, gZ_j \right\}$$

where n refers to a point in coordinate system 50 of the electric field based position system 18, g refers to a point in coordinate system 52 of the magnetic field based position system 20, and $j$=1, 2, ....N with N being the total number of planning positions. It should be understood that steps 62, 64 may be performed substantially simultaneously. It should also be understood that the steps may be performed for multiple electrical and magnetic position sensors 44, 46 on catheter 14. In some devices, multiple electrical position sensors 44 may be disposed proximate to a single magnetic position sensor 46 thereby resulting in the generation of a plurality of electrical planning positions for each magnetic planning position. In such cases, the method may further include the step 66 of averaging the planning positions for the electrical position sensors 44 disposed proximate the magnetic position sensor 46. The determination of planning positions for sensors 44, 46 on catheter 14 may generate more positions than required or desired. Therefore, the method may further include the step 68 of comparing a distance between a pair of planning positions for either a single sensor (e.g., electrical position sensor $44_1$ or magnetic position sensor $46_1$) or multiple sensors (e.g., electrical position sensors $44_1$ and $44_2$) and the step 70 of discarding one of the planning positions if the distance meets a predetermined characteristic relative to a predetermined threshold (e.g., is less than a predetermined distance such as 4.0 millimeters).

Once the planning positions are obtained, the planning stage may continue with the steps 72, 74 of computing mapping functions responsive to the planning positions for the electrical and magnetic position sensors $44_1$, $46_1$. One mapping function correlates the planning positions for the electrical position sensor $44_1$ and the magnetic position sensor $46_1$ such that the mapping function generates a mapped position for the electrical position sensor $44_1$ in the coordinate system 52 of the magnetic-field based positioning system 20 responsive to a position of the electrical position sensor in the coordinate system 50 of the electric-field based positioning system 18. The mapping function may be computed using radial basis function or thin plate spline interpolation with the basis function chosen as $r$ and a non-zero stiffness parameter $\lambda$ so that the registration between the coordinate systems 50, 52 is not rigid and the mapping is smooth without over-fitting noisy measurements. The basis function used for interpolation is selected from a range of the radial basis functions. One possible choice for the set of functions is:

$$\psi_i\left( \overrightarrow{nX} \right) = \left| \overrightarrow{nX} - \overrightarrow{nX_i} \right|$$

Where $\overrightarrow{nX}$ is the vector notation for the planning position {$nX_j$, $nY_j$, $nZ_j$} (similarly, as referenced hereinbelow, $\overrightarrow{gX}$ will represent the vector notation for the planning position {$gX_j$, $gY_j$, $gZ_j$}) and each function has a center at the corresponding point $\overrightarrow{nX_i}$. The right hand side of the above equation may be expressed in standard notation as:

$$\left| \vec{x} - \vec{x_i} \right| = \sqrt{\left( x - x_i \right)^2 + \left( y - y_i \right)^2 + \left( z - z_i \right)^2}$$

A map from coordinate system 50 to coordinate system 52 may be represented by the following functions:

$$gX(nX,nY,nZ) = b_1^x nX + b_2^x nY + b_3^x nZ + c^x + \sum_{j=1}^{N} a_j^x \psi_j \left( \overrightarrow{nX} \right)$$

$$gY(nX,nY,nZ) = b_1^y nX + b_2^y nY + b_3^y nZ + c^y + \sum_{j=1}^{N} a_j^y \psi_j \left( \overrightarrow{nX} \right)$$

$$gZ(nX,nY,nZ) = b_1^z nX + b_2^z nY + b_3^z nZ + c^z + \sum_{j=1}^{N} a_j^z \psi_j \left( \overrightarrow{nX} \right)$$

Where $a_j^x$, $a_j^y$, $a_j^z$, $b_j^x$, $b_j^y$, $b_j^z$ and $c^x, c^y, c^z$, j=1, 2....N, and i=1, 2, 3 are parameters chosen such that the planned positions are mapped as close as possible to measured positions and the degree of closeness is controlled by the smoothing parameter $\lambda$. The following equations then define the unknown parameters by providing a system of linear equations for a similar number of unknown parameters:

$$gX_i = b_1^x nX_i + b_2^x nY_i + b_3^x nZ_i + c^x + \sum_{j=1}^{N} a_j^x \left( \psi_j \left( \overrightarrow{nX_i} \right) - \lambda \delta_{ij} \right)$$

$$gY_i = b_1^y nX_i + b_2^y nY_i + b_3^y nZ_i + c^y + \sum_{j=1}^{N} a_j^y \left( \psi_j \left( \overrightarrow{nX_i} \right) - \lambda \delta_{ij} \right)$$

$$gZ_i = b_1^z nX_i + b_2^z nY_i + b_3^z nZ_i + c^z + \sum_{j=1}^{N} a_j^z \left( \psi_j \left( \overrightarrow{nX_i} \right) - \lambda \delta_{ij} \right)$$

$$\sum_{j=1}^{N} a_j^k = 0, \quad \sum_{j=1}^{N} a_j^k nX_j = 0, \quad \sum_{j=1}^{N} a_j^k nY_j = 0, \quad \sum_{j=1}^{N} a_j^k nZ_j = 0, \quad k = x, y, z,$$

Where $\delta_{ij}$ are the Kronecker symbols defined by the following equations:

$$\delta_{ij} = 1, \quad i = j$$

$$\delta_{ij} = 0, \quad i \neq j$$

The solution to these equations can be used to define the mapping function $gFS$ which can be applied as follows to map a position in coordinate system 50 into coordinate system 52:

$$\overrightarrow{gX} = gFS \left( \overrightarrow{nX} \right)$$

Further information regarding the above steps may be found in U.S. patent application publication US 2013/0066193 A1.

The mapping function $gFS$ may be used to detect shift or drift in the readings obtained by electric-field based positioning system 18 by applying the function to a position measurement for an electric position sensor $44_1$ as follows:

$$\overrightarrow{gX}_{44_1} = gFS \left( \overrightarrow{nX}_{44_1} \right)$$

The result can be compared against the position measurement for a corresponding magnetic sensor $46_1$ in coordinate

system 52 to obtain the required correction:

$$\overrightarrow{g\Delta} = \overrightarrow{gX_{46_1}} - \overrightarrow{gX_{44_1}}$$

This correction can then be applied to correct the locations of each electrical position sensor 44 within coordinate system 52:

$$\overrightarrow{gX_{corr}^{k}} = \overrightarrow{g\Delta} + \overrightarrow{gX^{k}}$$

Because the mapping function *gFS* is highly non-linear, global homogenous shifts in coordinate system 50 may not be properly corrected by applying the function. In accordance with one aspect of the present teachings, therefore, the use of an inverse mapping function *IgFS* has been developed to compensate for drifts and shift directly within coordinate system 50 before mapping function *gFS* is applied. Accordingly, in step 74 a mapping function is computed that correlates the planning positions for the electrical position sensor $44_1$ and the magnetic position sensor $46_1$ such that the mapping function generates a mapped position for the magnetic position sensor $46_1$ in the coordinate system 50 of the electric-field based positioning system 18 responsive to a position of the magnetic position sensor $46_1$ in the coordinate system 52 of the magnetic-field based positioning system 20. The mapping function *IgFS* can be computed using equations similar to those used to compute the mapping function *gFS* by using the same basis functions and interchanging the magnetic and electrical planning positions. Thus, a map from coordinate system 52 to coordinate system 50 may be represented by the following functions:

$$nX(gX, gY, gZ) = \widetilde{b}_1^{\,x} gX + \widetilde{b}_2^{\,x} gY + \widetilde{b}_3^{\,x} gZ + \widetilde{c}^{\,x} + \sum_{j=1}^{N} \widetilde{a}_j^{\,x} \psi_j\left(\overrightarrow{gX}\right)$$

$$nY(gX, gY, gZ) = \widetilde{b}_1^{\,y} gX + \widetilde{b}_2^{\,y} gY + \widetilde{b}_3^{\,y} gZ + \widetilde{c}^{\,y} + \sum_{j=1}^{N} \widetilde{a}_j^{\,y} \psi_j\left(\overrightarrow{gX}\right)$$

$$nZ(gX, gY, gZ) = \widetilde{b}_1^{\,z} gX + \widetilde{b}_2^{\,z} gY + \widetilde{b}_3^{\,z} gZ + \widetilde{c}^{\,z} + \sum_{j=1}^{N} \widetilde{a}_j^{\,z} \psi_j\left(\overrightarrow{gX}\right)$$

Where $\widetilde{a}_j^{\,x}$, $\widetilde{a}_j^{\,y}$, $\widetilde{a}_j^{\,z}$, $\widetilde{b}_j^{\,x}$, $\widetilde{b}_j^{\,y}$, $\widetilde{b}_j^{\,z}$ and $\widetilde{c}^x, \widetilde{c}^y, \widetilde{c}^z$, j=1, 2....N, and i=1, 2, 3 are parameters chosen such that the planned positions are mapped as close as possible to measured positions and the degree of closeness is controlled by the smoothing parameter $\lambda$. The following equations then define the unknown parameters by providing a system of linear equations for a similar number of unknown parameters:

$$nX_i = \widetilde{b}_1^{\,x} gX_i + \widetilde{b}_2^{\,x} gY_i + \widetilde{b}_3^{\,x} gZ_i + \widetilde{c}^{\,x} + \sum_{j=1}^{N} \widetilde{a}_j^{\,x}\left(\psi_j\left(\overrightarrow{gX_i}\right) - \lambda\delta_{ij}\right)$$

$$nY_i = \widetilde{b}_1^{\,y} gX_i + \widetilde{b}_2^{\,y} gY_i + \widetilde{b}_3^{\,y} gZ_i + \widetilde{c}^{\,y} + \sum_{j=1}^{N} \widetilde{a}_j^{\,y}\left(\psi_j\left(\overrightarrow{gX_i}\right) - \lambda\delta_{ij}\right)$$

$$nZ_i = \widetilde{b}_1^{\,z} gX_i + \widetilde{b}_2^{\,z} gY_i + \widetilde{b}_3^{\,z} gZ_i + \widetilde{c}^{\,z} + \sum_{j=1}^{N} \widetilde{a}_j^{\,z}\left(\psi_j\left(\overrightarrow{gX_i}\right) - \lambda\delta_{ij}\right)$$

$$\sum_{j=1}^{N} \widetilde{a}_j^{\,k} = 0, \ \sum_{j=1}^{N} \widetilde{a}_j^{\,k} gX_j = 0, \ \sum_{j=1}^{N} \widetilde{a}_j^{\,k} gY_j = 0, \ \sum_{j=1}^{N} \widetilde{a}_j^{\,k} gZ_j = 0, \ \text{k=x, y, z,}$$

Where $\delta_{ij}$ are the Kronecker symbols defined by the following equations:

$$\delta_{ij} = 1, \quad i = j$$

$$\delta_{ij} = 0, \quad i \neq j$$

The solution to these equations can be used to define the mapping function *IgFS* which can be applied as follows to map a position in coordinate system 52 into coordinate system 50:

$$\overrightarrow{nX} = IgFS\left(\overrightarrow{gX}\right)$$

Although Figure 3 illustrates steps 72, 74 as occurring in succession, it should be understood that the order of steps 72, 74 could be reversed and or, in other embodiments the steps 72, 74 may be performed simultaneously.

Although the mapping function *IgFS* is intended to be the inverse of the mapping function *gFS,* the function is only an approximate inverse. Thus, sequential application of the mapping functions yields a position that is only an approximate of the original position:

$$\overrightarrow{nX} \approx IgFS\left(gFS\left(\overrightarrow{nX}\right)\right)$$

In order to make the mapping function *IgFS* closer to a true inverse of the mapping function *gFS,* the method may utilize additional planning positions and, in particular, virtual planning positions. Accordingly, the method may optionally include the steps 76, 78 of determining a virtual planning position for electrical position sensor $44_1$ within coordinate system 50 and a corresponding virtual planning position for magnetic position sensor $46_1$ within coordinate system 52. The virtual planning position for position sensor $44_1$ may be located at a boundary in coordinate system 50. The corresponding virtual planning position for position sensor $46_1$ could then be obtained by applying the mapping function *gFS* to the virtual planning position for position sensor $44_1$ (in this embodiment, it should be understood that the step 74 would follow step 72 sequentially). The virtual planning positions may approximate a grid pattern on a surface surrounding the planning positions determined in steps 62, 64. The surface may comprise the faces of an axis-aligned rectangular parallelepiped enclosing those planning positions. Alternatively, the virtual planning positions may also approximate the volume in which the planning positions reside if computational resources are sufficient. The number of virtual planning positions may be restricted to a number that is less than the number of planning positions obtained in steps 62, 64 for computational efficiency.

Once the mapping functions *gFS* and *IgFS* have been computed, an operating stage of the diagnostic or treatment procedure may commence through which catheter 14 or another medical device may be maneuvered within body 12 to a region of interest. The method may therefore continue with the step 80 of determining an operating position for electrical position sensor $44_1$ within coordinate system 50 of electric field based positioning system 18 and the step 82 of determining an operating position for magnetic position sensor $46_1$, disposed proximate position sensor $44_1$, within coordinate system 52 of magnetic field based positioning system 20. The method may then proceed with the step 84 of applying mapping function *IgFS* to generate a mapped position for magnetic position sensor $46_1$ in coordinate system 50 responsive to the operating position of magnetic position sensor $46_1$ in coordinate system 52. Once the mapped position is obtained, the method may continue with the step 86 of determining an adjusted or corrected operating position for electrical position sensor $44_1$ in coordinate system 50 responsive to the mapped position of magnetic position sensor $46_1$. In particular, step 86 may include the substep 88 of determining a difference between the operating position for electrical position sensor $44_1$ in coordinate system 50 and the mapped position for magnetic position sensor $46_1$ in coordinate system 50:

$$\overrightarrow{\Delta_1} = IgFS\left(\overrightarrow{g46_1}\right) - \overrightarrow{n44_1}$$

Step 86 may further include the substep 90 of modifying the operating position for electrical position sensor $44_1$ in

coordinate system 50 by the difference to obtain the adjusted or corrected operating position of electrical position sensor $44_1$ in coordinate system 50:

$$\overrightarrow{n44_{1corr}} = \overrightarrow{n44_1} + \overrightarrow{\Delta_1}$$

The adjusted operating position thereby accounts for drift and shift resulting from changes in impedance in body 12 by correlating the position of electrical position sensor $44_1$ with the position of magnetic position sensor $46_1$ in coordinate system 50. Because the position of magnetic position sensor $46_1$ in coordinate system 50 as detected by magnetic positioning system 20 is not subject to the changes in impedance, mapping function *IgFS* produces a stable reference in coordinate system 50 that can be used to correct for drift and shifts in the detected position of electrical position sensor $44_1$ in coordinate system 50.

The above-described embodiment contemplates the use of a single magnetic position sensor $46_1$ as a positional reference for electrical position sensor $44_1$. If catheter 14 is equipped with multiple magnetic sensors $46_1$, $46_2$, however, or if multiple catheters having magnetic position sensors 46 are employed in body 12 at the same time, correction factors based on each magnetic position sensor 46 can be obtained and used to obtain the adjusted operation position for electrical position sensor $44_1$. Therefore, another embodiment of a method in accordance with the present teachings may recursively apply steps 82, 84, 86 (including substeps 88, 90) for each magnetic position sensor 46. Thus, the method may include the step of determining an operating position for another magnetic position sensor disposed proximate electrical position sensor $44_1$ such as magnetic position sensor $46_2$ on catheter 14 within coordinate system 52. The method may further include the step of applying mapping function *IgFS* to generate a mapped position for magnetic position sensor $46_2$ in coordinate system 50 responsive to the operating position of magnetic position sensor $46_2$. The method may continue with the step of determining an adjusted operating position for electrical position sensor $44_1$ in coordinate system 50 responsive to the mapped position of magnetic position sensor $46_2$. In particular, a difference may again be determined between the operating position for electrical position sensor $44_1$ in coordinate system 50 and the mapped position for magnetic position sensor $46_2$ in coordinate system 50:

$$\overrightarrow{\Delta_2} = IgFS\left(\overrightarrow{g46_2}\right) - \overrightarrow{n44_1}$$

Thereafter, the operating position for electrical position sensor $44_1$ in coordinate system 50 may be modified by the difference to obtain the adjusted or corrected operating position of electrical position sensor $44_1$ in coordinate system 50:

$$\overrightarrow{n44_{1corr}} = \overrightarrow{n44_1} + \overrightarrow{\Delta_2}$$

After determining an adjusted or corrected operating position for electrical position sensor $44_1$ in coordinate system 50 responsive to the mapped positions of each magnetic position sensor 46, the method may continue with the step 92 of determining a total adjusted operating position for electrical position sensor $44_1$ in coordinate system 50. In the case of a single magnetic sensor 46, the total adjusted operating position will simply be the same as the adjusted operating position obtained based on that magnetic sensor. In the case of multiple magnetic position sensors 46, the total adjusted operating position may be calculated in various ways. In one embodiment, step 92 includes a substep 94 of averaging the adjusted operating positions. In another embodiment, step 92 includes the substep 94 of weighting each of the adjusted operating positions responsive to actual distances of electrical position sensor $44_1$ relative to the magnetic position sensors $46_1$, $46_2$ such that, for example, the adjusted operating position obtained in response to magnetic position sensor $46_1$, which is closer to electrical position sensors $44_1$, is accorded more weight than the adjusted operating position obtained in response to magnetic position sensor $46_2$. In yet another embodiment, the adjusted operating positions are weighted based on the distance of the operating position for each magnetic position sensors $46_1$, $46_2$, from the planning positions measured earlier in order to account for potential decreases in accuracy in mapping functions *gFS* and *IgFS* as one moves further from the reference points used to construct the functions. The distance determination can be made in a variety of ways known in the art. In one embodiment, the magnetic space is divided into a grid of cells each of which contains one of the planning positions or is empty. Once an operating position for sensor $46_1$, $46_2$, is known, a corresponding cell can be identified and surrounding cells examined to identify the distance to the nearest planning position. In order to avoid abrupt changes or discontinuities, the weighting for adjusted operating positions based on sensors that are relatively far away from planning positions may be gradually adjusted (increased or decreased) over time instead of being simply proportional to distance. Thus, if a magnetic position sensor 46 moves abruptly, is removed from body 14 or is disabled in some way, its contribution to the total adjusted operating position is gradually adjusted rather than causing a sudden shift. In the case where the only or final magnetic sensor 46 becomes disabled or leaves the magnetic field, its last known operating position is retained for used in obtaining the adjusted or corrected operating position for electrical position sensor $44_1$ in coordinate system 50. If the sensor 46 becomes enabled or otherwise returns to the magnetic field, a gradual transition is made from the last known operating position to the current operating position.

The method may continue with the step 98 of applying mapping function *gFS* to generate a mapped position for electrical position sensor $44_1$ in coordinate system 52 responsive to the adjusted operating position $\overrightarrow{n44_{1corr}}$ for electrical position sensor $44_1$ in coordinate system 50:

$$\overrightarrow{g44_1} = gFS\left(\overrightarrow{n44_{1corr}}\right)$$

Finally, the method may also include the step 100 of displaying an image corresponding to electrical position sensor $44_1$ on display 22 responsive to the adjusted operating position $\overrightarrow{n44_{1corr}}$ by, for example, using the value acquired in step 94.

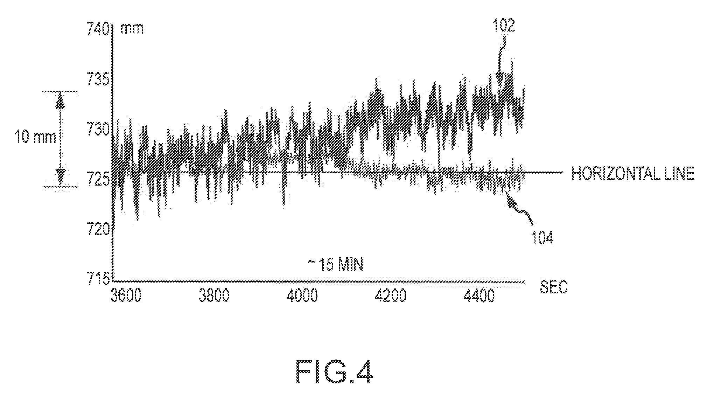

Referring now to Figure 4, the impact of the system 10 and method is illustrated. The system 10 and method were tested using position data obtained for electrical position sensors 44 and magnetic position sensors 46 using the systems made available under the trademark "ENSITE NAVX" by St. Jude Medical, Inc. and under the trademark "GMPS" by MediGuide, Ltd., respectively. Figure 4 illustrates a trace 102 for the position of one electrical position sensor 44 along one axis disposed proximate a magnetic position sensor 46 as determined without use of the system and method. Figure 4 further illustrates a trace 104 for the position of the same position sensor 44 adjusted or corrected using the system 10 and method described herein. As illustrated in Figure 4, use of the system and method reduced the variability in the original trace 102 and removed much of the drift in the signal.

A system 10 and method for navigating a medical device within a body 12 in accordance with the present teachings enables consistent correction or errors in position measurements due to shift or drift in patient impedance levels. Further, the system 10 and method do not require the use of an additional reference catheter and the resulting increases in procedure time and risks.

Although several embodiments of this invention have been described above with a certain degree of particularity, those skilled in the art could make numerous alterations to the disclosed embodiments without departing from the scope of this invention. All directional references (e.g., upper, lower, upward, downward, left, right, leftward, rightward, top, bottom, above, below, vertical, horizontal, clockwise and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not as limiting. Changes in detail or structure may be made without departing from the invention as defined in the appended claims.

**Claims**

1.  A system for navigating a medical device within a body, comprising:

    an electronic control unit (24) configured to:

    determine an operating position for a first electrical position sensor ($44_1$) on said medical device within a first coordinate system, said first coordinate system defined by an electric field based positioning system (18); determine an operating position for a first magnetic position sensor ($46_1$) on said medical device within a second coordinate system, said second coordinate system defined by a magnetic field based positioning system (20), said first magnetic position sensor ($46_1$) disposed proximate said first electrical position sensor ($44_1$);
    apply a first mapping function correlating said operating positions of said first electrical position sensor ($44_1$) and said first magnetic position sensor ($46_1$), said first mapping function generating a mapped position for said first magnetic position sensor ($46_1$) in said first coordinate system responsive to said operating position of said first magnetic position sensor ($46_1$) in said second coordinate system; and,
determine a first adjusted operating position for said first electrical position sensor ($44_1$) in said first coordinate system responsive to said mapped position of said first magnetic position sensor ($46_1$).

2. The system of claim 1 wherein said electronic control unit (24) is further configured to apply a second mapping function correlating said operating positions of said first electrical position sensor ($44_1$) and said first magnetic position sensor ($46_1$), said second mapping function generating a mapped position for said first electrical position sensor ($44_1$) in said second coordinate system responsive to said first adjusted operating position for said first electrical position sensor ($44_1$) in said first coordinate system.

3. The system of claim 1 wherein said electronic control unit (24) is further configured to:

determine a planning position for said first electrical position sensor ($44_1$) within said first coordinate system;
determine a planning position for said first magnetic position sensor ($46_1$) within said second coordinate system; and,
compute said first mapping function responsive to said planning positions for said first electrical position sensor ($44_1$) and said first magnetic position sensor ($46_1$).

4. The system of claim 3 wherein said electronic control unit (24) is further configured to:

determine a virtual planning position for said first electrical position sensor ($44_1$) within said first coordinate system, said virtual planning position located at a boundary in said first coordinate system; and,
determine a virtual planning position for said first magnetic position sensor ($46_1$) within said second coordinate system, said virtual planning position for said first magnetic sensor corresponding to said virtual planning position for said first electrical position sensor ($44_1$);
wherein said first mapping function is computed responsive to said virtual planning positions for said first electrical position sensor ($44_1$) and said first magnetic position sensor ($46_1$).

5. The system of claim 3 wherein said electronic control unit (24) is further configured to compute a second mapping function responsive to said planning positions for said first electrical position sensor ($44_1$) and said first magnetic position sensor ($46_1$), said second mapping function correlating said planning positions of said first electrical position sensor ($44_1$) and said first magnetic position sensors ($46_1$), said second mapping function generating a mapped position for said first electrical position sensor ($44_1$) in said second coordinate system responsive to said planning position of said first electrical position sensor ($44_1$) in said first coordinate system.

6. The system of claim 3 wherein said electronic control unit (24) is further configured to:

determine a planning position for a second electrical position sensor ($44_2$) on said medical device within said first coordinate system, said second electrical position sensor ($44_2$) disposed proximate said first magnetic position sensor ($46_1$); and,
average said planning positions of said first ($44_1$) and second electrical position sensors ($44_2$) before computing said first mapping function.

7. The system of claim 3 wherein said electronic control unit (24) is further configured to:

determine a planning position for one of a second electrical position sensor ($44_2$) within said first coordinate system and a second magnetic position sensor ($46_2$) within said second coordinate system;
compare a distance between said planning position of said one position sensor and said planning position of a corresponding one of said first electrical position sensor ($44_1$) and said first magnetic position sensor ($46_1$); and,
discard said planning position for said one position sensor if said distance meets a predetermined characteristic relative to a predetermined threshold.

8. The system of claim 1 wherein said electronic control unit (24) is further configured, in determining said first adjusted operating position, to:

determine a difference between said operating position for said first electrical position sensor ($44_1$) and said mapped position for said first magnetic position sensor ($46_1$); and,
modify said operating position for said first electrical position sensor ($44_1$) by said difference to obtain said first adjusted operating position.

9. The system of claim 1 wherein said electronic control unit (24) is further configured to:

determine an operating position for a second magnetic position sensor ($46_2$) on said medical device within said second coordinate system, said second magnetic position sensor ($46_2$) disposed proximate said first electrical position sensor ($44_1$);
apply a second mapping function correlating said operating positions of said first electrical position sensor ($44_1$) and said second magnetic position sensor ($46_2$), said second mapping function generating a mapped position for said second magnetic position sensor ($46_2$) in said first coordinate system responsive to said operating position of said second magnetic position sensor ($46_2$) in said second coordinate system;
determine a second adjusted operating position for said first electrical position sensor ($44_1$) in said first coordinate system responsive to said mapped position of said second magnetic position sensor; and,
determine a total adjusted operating position for said first electrical position sensor ($44_1$) in said first coordinate system responsive to said first and second adjusted operating positions.

10. The system of claim 9 wherein said electronic control unit (24) is further configured, in determining said total adjusted operating position, to average said first and second adjusted operating positions.

11. The system of claim 9 wherein said electronic control unit (24) is further configured, in determining said total adjusted operating position, to weight each of said first and second adjusted operating positions responsive to actual distances of said first electrical position sensor ($44_1$) relative to said first magnetic position sensor ($46_1$) and said second magnetic position sensor ($46_2$).

12. The system of claim 1 wherein said electronic control unit (24) is further configured to display an image corresponding to said first electrical position sensor ($44_1$) on a display responsive to said first adjusted operating position.

13. A method for navigating a medical device within a body, comprising:

determining an operating position for a first electrical position sensor ($44_1$) on said medical device within a first coordinate system, said first coordinate system defined by an electric field based positioning system (18);
determining an operating position for a first magnetic position sensor ($46_1$) on said medical device within a second coordinate system, said second coordinate system defined by a magnetic field based positioning system, said first magnetic position sensor ($46_1$) disposed proximate said first electrical position sensor ($44_1$);
applying a first mapping function correlating said operating positions of said first electrical position sensor ($44_1$) and said first magnetic position sensor ($46_1$), said first mapping function generating a mapped position for said first magnetic position sensor ($46_1$) in said first coordinate system responsive to said operating position of said first magnetic position sensor ($46_1$) in said second coordinate system; and,
determining a first adjusted operating position for said first electrical position sensor ($44_1$) in said first coordinate system responsive to said mapped position of said first magnetic position sensor ($46_1$).

**Patentansprüche**

1. System zum Navigieren einer medizinischen Vorrichtung innerhalb eines Körpers, mit: einer elektronischen Steuerungseinheit (24), die ausgebildet ist zum:

Bestimmen einer Betriebsposition für einen ersten elektrischen Positionssensor ($44_1$) auf der medizinischen Vorrichtung innerhalb eines ersten Koordinatensystems, wobei das erste Koordinatensystem durch ein elektrisches Feld definiert ist, basierend auf einem Positionsbestimmungssystem (18);
Bestimmen einer Betriebsposition für einen ersten magnetischen Positionssensor ($46_1$) auf der medizinischen Vorrichtung innerhalb eines zweiten Koordinatensystems, wobei das zweite Koordinatensystem durch ein Magnetfeld definiert ist, basierend auf einem Positionsbestimmungssystem (20), wobei der erste magnetische Positionssensor ($46_1$) sich benachbart zu dem ersten elektrischen Positionssensor ($44_1$) befindet;
Anwenden einer ersten Abbildungsfunktion, die die Betriebspositionen des ersten elektrischen Positionssensors ($44_1$) und des ersten magnetischen Positionssensors ($46_1$) korreliert, wobei die erste Abbildungsfunktion eine abgebildete Position für den ersten magnetischen Positionssensor ($46_1$) in dem ersten Koordinatensystem erzeugt in Reaktion auf die Betriebsposition des ersten magnetischen Positionssensors ($46_1$) in dem zweiten

Koordinatensystem; und

Bestimmen einer ersten eingestellten Betriebsposition für den ersten elektrischen Positionssensor ($44_1$) in dem ersten Koordinatensystem in Reaktion auf die abgebildete Position des ersten magnetischen Positionssensors ($46_1$).

2. System nach Anspruch 1, bei dem die elektronische Steuerungseinheit (24) ferner ausgebildet ist zum Anwenden einer zweiten Abbildungsfunktion, die die Betriebspositionen des ersten elektrischen Positionssensors ($44_1$) und des ersten magnetischen Positionssensors ($46_1$) korreliert, wobei die zweite Abbildungsfunktion eine abgebildete Position für den ersten elektrischen Positionssensor ($44_1$) in dem zweiten Koordinatensystem erzeugt in Reaktion auf die erste eingestellte Betriebsposition für den ersten elektrischen Positionssensor ($44_1$) in dem ersten Koordinatensystem.

3. System nach Anspruch 1, bei dem die elektronische Steuerungseinheit (24) ferner ausgebildet ist zum:

Bestimmen einer Planungsposition für den ersten elektrischen Positionssensor ($44_1$) innerhalb des ersten Koordinatensystems;
Bestimmen einer Planungsposition für den ersten magnetischen Positionssensor ($46_1$) innerhalb des zweiten Koordinatensystems; und
Berechnen der ersten Abbildungsfunktion in Reaktion auf die Planungspositionen für den ersten elektrischen Positionssensor ($44_1$) und den ersten magnetischen Positionssensor ($46_1$).

4. System nach Anspruch 3, bei dem die elektronische Steuerungseinheit (24) ferner ausgebildet ist zum:

Bestimmen einer virtuellen Planungsposition für den ersten elektrischen Positionssensor ($44_1$) innerhalb des ersten Koordinatensystems, wobei die virtuelle Planungsposition sich an einer Grenze in dem ersten Koordinatensystem befindet; und
Bestimmen einer virtuellen Planungsposition für den ersten magnetischen Positionssensor ($46_1$) innerhalb des zweiten Koordinatensystems, wobei die virtuelle Planungsposition für den ersten magnetischen Sensor der virtuellen Planungsposition für den ersten elektrischen Positionssensor ($44_1$) entspricht;
wobei die erste Abbildungsfunktion berechnet wird in Reaktion auf die virtuellen Planungspositionen für den ersten elektrischen Positionssensor ($44_1$) und den ersten magnetischen Positionssensor ($46_1$).

5. System nach Anspruch 3, bei dem die elektronische Steuerungseinheit (24) ferner ausgebildet ist zum Berechnen einer zweiten Abbildungsfunktion in Reaktion auf die Planungspositionen des ersten elektrischen Positionssensors ($44_1$) und des ersten magnetischen Positionssensors ($46_1$), wobei die zweite Abbildungsfunktion die Planungspositionen des ersten elektrischen Positionssensors ($44_1$) und des ersten magnetischen Positionssensors ($46_1$) korreliert, wobei die zweite Abbildungsfunktion eine abgebildete Position für den ersten elektrischen Positionssensor ($44_1$) in dem zweiten Koordinatensystem erzeugt in Reaktion auf die Planungsposition des ersten elektrischen Positionssensors ($44_1$) in dem ersten Koordinatensystem.

6. System nach Anspruch 3, bei dem die elektronische Steuerungseinheit (24) ferner ausgebildet ist zum:

Bestimmen einer Planungsposition für einen zweiten elektrischen Positionssensor ($44_2$) auf der medizinischen Vorrichtung innerhalb des ersten Koordinatensystems, wobei der zweite elektrische Positionssensor ($44_2$) sich benachbart zu dem ersten magnetischen Positionssensor ($46_1$) befindet; und
Mitteln der Planungspositionen des ersten ($44_1$) und des zweiten elektrischen Positionssensors ($44_2$), bevor die erste Abbildungsfunktion gerechnet wird.

7. System nach Anspruch 3, bei dem die elektronische Steuerungseinheit (24) ferner ausgebildet ist zum:

Bestimmen einer Planungsposition für einen von einem zweiten elektrischen Positionssensor ($44_2$) in dem ersten Koordinatensystem und einem zweiten magnetischen Positionssensor ($46_2$) in dem zweiten Koordinatensystem;
Vergleichen eines Abstands zwischen der Planungsposition des einen Positionssensors und der Planungsposition eines Entsprechenden von dem ersten elektrischen Positionssensor ($44_1$) und dem ersten magnetischen Positionssensor ($46_1$); und
Verwerfen der Planungsposition für den einen Positionssensor, wenn der Abstand eine vorbestimmte Charakteristik relativ zu einem vorbestimmten Schwellenwert verfüllt.

**8.** System nach Anspruch 1, bei dem die elektronische Steuerungseinheit (24) ferner ausgebildet ist, beim Bestimmen der ersten eingestellten Betriebsposition, zum:

Bestimmen einer Differenz zwischen der Betriebsposition für den ersten elektrischen Positionssensor ($44_1$) und der abgebildeten Position für den ersten magnetischen Positionssensor ($46_1$); und
Modifizieren der Betriebsposition für den ersten elektrischen Positionssensor ($44_1$) durch die Differenz, um die erste eingestellte Betriebsposition zu erhalten.

**9.** System nach Anspruch 1, bei dem die elektronische Steuerungseinheit (24) ferner ausgebildet zum:

Bestimmen einer Betriebsposition für einen zweiten magnetischen Positionssensor ($46_2$) auf der medizinischen Vorrichtung innerhalb des zweiten Koordinatensystems, wobei der zweite magnetische Positionssensor ($46_2$) sich benachbart zu dem ersten elektrischen Positionssensor ($44_1$) befindet;
Anwenden einer zweiten Abbildungsfunktion, die die Betriebspositionen des ersten elektrischen Positionssensors ($44_1$) und des zweiten magnetischen Positionssensors ($46_2$) korreliert, wobei die zweite Abbildungsfunktion eine abgebildete Position für den zweiten magnetischen Positionssensor ($46_2$) in dem ersten Koordinatensystem erzeugt in Reaktion auf die Betriebsposition des zweiten magnetischen Positionssensors ($46_2$) in dem zweiten Koordinatensystem;
Bestimmen einer zweiten eingestellten Betriebsposition für den ersten elektrischen Positionssensor ($44_1$) in dem ersten Koordinatensystem in Antwort auf die abgebildete Position des zweiten magnetischen Positionssensors; und
Bestimmen einer vollständig eingestellten Betriebsposition für den ersten elektrischen Positionssensor ($44_1$) in dem ersten Koordinatensystem in Reaktion auf die erste und zweite eingestellte Betriebsposition.

**10.** System nach Anspruch 9, bei dem die elektronische Steuerungseinheit (24) ferner ausgebildet ist zum Mitteln der ersten und zweiten eingestellten Betriebsposition bei dem Bestimmen der vollständig eingestellten Betriebsposition.

**11.** System nach Anspruch 9, bei dem die elektronische Steuerungseinheit (24) ferner ausgebildet ist, beim Bestimmen der vollständig eingestellten Betriebsposition, zum Gewichten von jeder von der ersten und zweiten eingestellten Betriebsposition in Reaktion auf tatsächliche Abstände des ersten elektrischen Positionssensors ($44_1$) relativ zu dem ersten magnetischen Positionssensor ($46_1$) und dem zweiten magnetischen Positionssensor ($46_2$).

**12.** System nach Anspruch 1, bei dem die elektronische Steuerungseinheit (24) ferner ausgebildet ist zum Anzeigen eines Bilds, das dem ersten elektrischen Positionssensor ($44_1$) entspricht, auf einer Anzeige in Reaktion auf die erste eingestellte Betriebsposition.

**13.** Verfahren zum Navigieren einer medizinischen Vorrichtung in einem Körper, mit:

Bestimmen einer Betriebsposition für einen ersten elektronischen Positionssensor ($44_1$) auf der medizinischen Vorrichtung in einem ersten Koordinatensystem, wobei das erste Koordinatensystem definiert ist durch ein elektrisches Feld, basierend auf einem Positionsbestimmungssystem (18);
Bestimmen einer Betriebsposition für einen ersten magnetischen Positionssensor ($46_1$) auf der medizinischen Vorrichtung in einem zweiten Koordinatensystem, wobei das zweite Koordinatensystem durch ein Magnetfeld definiert ist, basierend auf einem Positionsbestimmungssystem, wobei der erste magnetische Positionssensor ($46_1$) sich benachbart zu dem ersten elektrischen Positionssensor ($44_1$) befindet;
Anwenden einer ersten Abbildungsfunktion, die die Betriebspositionen des ersten elektrischen Positionssensors ($44_1$) und des ersten magnetischen Positionssensors ($46_1$) korreliert, wobei die erste Abbildungsfunktion eine abgebildete Position für den ersten magnetischen Positionssensor ($46_1$) in dem ersten Koordinatensystem erzeugt in Reaktion auf die Betriebsposition des ersten magnetischen Positionssensors ($46_1$) in dem zweiten Koordinatensystem; und
Bestimmen einer ersten eingestellten Betriebsposition für den ersten elektrischen Positionssensor ($44_1$) in dem ersten Koordinatensystem in Reaktion auf die abgebildete Position des ersten magnetischen Positionssensors ($46_1$).

**Revendications**

**1.** Système pour faire naviguer un dispositif médical à l'intérieur d'un corps, comprenant:

une unité de commande électronique (24) configurée pour:

déterminer une position opérationnelle pour un premier capteur de position électrique ($44_1$) sur ledit dispositif médical à l'intérieur d'un premier système de coordonnées, ledit premier système de coordonnées étant défini par un système de positionnement basé sur un champ électrique (18);

déterminer une position opérationnelle pour un premier capteur de position magnétique ($46_1$) sur ledit dispositif médical à l'intérieur d'un deuxième système de coordonnées, ledit deuxième système de coordonnées étant défini par un système de positionnement basé sur un champ magnétique (20), ledit premier capteur de position magnétique ($46_1$) étant disposé à proximité dudit premier capteur de position électrique ($44_1$);

appliquer une première fonction de mappage pour corréler lesdites positions opérationnelles dudit premier capteur de position électrique ($44_1$) et dudit premier capteur de position magnétique ($46_1$), ladite première fonction de mappage générant une position mappée pour ledit premier capteur de position magnétique ($46_1$) dans ledit premier système de coordonnées en réponse à ladite position opérationnelle dudit premier capteur de position magnétique ($46_1$) dans ledit deuxième système de coordonnées; et

déterminer une première position opérationnelle ajustée pour ledit premier capteur de position électrique ($44_1$) dans ledit premier système de coordonnées en réponse à ladite position mappée pour ledit premier capteur de position magnétique ($46_1$).

2. Système selon la revendication 1, dans lequel ladite unité de commande électronique (24) est en outre configurée pour appliquer une deuxième fonction de mappage pour corréler lesdites positions opérationnelles dudit premier capteur de position électrique ($44_1$) et dudit premier capteur de position magnétique ($46_1$), ladite deuxième fonction de mappage générant une position mappée pour ledit premier capteur de position électrique ($44_1$) dans ledit deuxième système de coordonnées en réponse à ladite position opérationnelle ajustée dudit premier capteur de position électrique ($44_1$) dans ledit premier système de coordonnées.

3. Système selon la revendication 1, dans lequel ladite unité de commande électronique (24) est en outre configurée pour:

déterminer une position de planification pour ledit premier capteur de position électrique ($44_1$) à l'intérieur dudit premier système de coordonnées;

déterminer une position de planification pour ledit premier capteur de position magnétique ($46_1$) à l'intérieur dudit deuxième système de coordonnées; et

calculer ladite première fonction de mappage en réponse auxdites positions de planification pour ledit premier capteur de position électrique ($44_1$) et ledit premier capteur de position magnétique ($46_1$).

4. Système selon la revendication 3, dans lequel ladite unité de commande électronique (24) est en outre configurée pour:

déterminer une position de planification virtuelle pour ledit premier capteur de position électrique ($44_1$) à l'intérieur dudit premier système de coordonnées, ladite position de planification virtuelle étant située à une frontière dans ledit premier système de coordonnées; et

déterminer une position de planification virtuelle pour ledit premier capteur de position magnétique ($46_1$) à l'intérieur dudit deuxième système de coordonnées, ladite position de planification virtuelle pour ledit premier capteur magnétique correspondant à ladite position de planification virtuelle pour ledit premier capteur de position électrique ($44_1$),

dans lequel ladite première fonction de mappage est calculée en réponse auxdites positions de planification virtuelles pour ledit premier capteur de position électrique ($44_1$) et ledit premier capteur de position magnétique ($46_1$).

5. Système selon la revendication 3, dans lequel l'unité de commande électronique (24) est en outre configurée pour calculer une deuxième fonction de mappage en réponse auxdites positions de planification pour ledit premier capteur de position électrique ($44_1$) et ledit premier capteur de position magnétique ($46_1$), ladite deuxième fonction de mappage corrélant lesdites positions de planification dudit premier capteur de position électrique ($44_1$) et dudit premier capteur de position magnétique ($46_1$), ladite deuxième fonction de mappage générant une position mappée pour ledit premier capteur de position électrique ($44_1$) dans ledit deuxième système de coordonnées en réponse à ladite position de planification dudit premier capteur de position électrique ($44_1$) dans ledit premier système de coordonnées.

**6.** Système selon la revendication 3, dans lequel ladite unité de commande électronique (24) est en outre configurée pour:

déterminer une position de planification pour un deuxième capteur de position électrique ($44_2$) sur ledit dispositif médical à l'intérieur dudit premier système de coordonnées, ledit deuxième capteur de position électrique ($44_2$) étant disposé à proximité dudit premier capteur de position magnétique ($46_1$); et

calculer la moyenne desdites positions de planification desdits premier ($44_1$) et deuxième ($44_2$) capteurs de position électrique avant le calcul de ladite première fonction de mappage.

**7.** Système selon la revendication 3, dans lequel ladite unité de commande électronique (24) est en outre configurée pour:

déterminer une position de planification pour un parmi un deuxième capteur de position électrique ($44_2$) à l'intérieur dudit premier système de coordonnées et un deuxième capteur de position magnétique ($46_2$) à l'intérieur dudit deuxième système de coordonnées;

comparer une distance entre ladite position de planification dudit un capteur de position et ladite position de planification d'un correspondant parmi ledit premier capteur de position électrique ($44_1$) et ledit premier capteur de position magnétique ($46_1$); et

rejeter ladite position de planification pour ledit un capteur de position si ladite distance rencontre une caractéristique prédéterminée relative à un seuil prédéterminé.

**8.** Système selon la revendication 1, dans lequel ladite unité de commande électronique (24) est en outre configurée, lors de la détermination de ladite première position opérationnelle ajustée, pour:

déterminer une différence entre ladite position opérationnelle pour ledit premier capteur de position électrique ($44_1$) et ladite position mappée pour ledit premier capteur de position magnétique ($46_1$); et

modifier ladite position opérationnelle pour ledit premier capteur de position électrique ($44_1$) par ladite différence afin d'obtenir ladite première position opérationnelle ajustée.

**9.** Système selon la revendication 1, dans lequel ladite unité de commande électronique (24) est en outre configurée pour:

déterminer une position opérationnelle pour un deuxième capteur de position magnétique ($46_2$) sur ledit dispositif médical à l'intérieur dudit deuxième système de coordonnées, ledit deuxième capteur de position magnétique ($46_2$) étant disposé à proximité dudit premier capteur de position électrique ($44_1$);

appliquer une deuxième fonction de mappage pour corréler lesdites positions opérationnelles dudit premier capteur de position électrique ($44_1$) et dudit deuxième capteur de position magnétique ($46_2$), ladite deuxième fonction de mappage générant une position mappée pour ledit deuxième capteur de position magnétique ($46_2$) dans ledit premier système de coordonnées en réponse à ladite position opérationnelle dudit deuxième capteur de position magnétique ($46_2$) dans ledit deuxième système de coordonnées;

déterminer une deuxième position opérationnelle ajustée pour ledit premier capteur de position électrique ($44_1$) dans ledit premier système de coordonnées en réponse à ladite position mappée pour ledit deuxième capteur de position magnétique; et

déterminer une position opérationnelle ajustée totale pour ledit premier capteur de position électrique ($44_1$) dans ledit premier système de coordonnées en réponse auxdites première et deuxième positions opérationnelles ajustées.

**10.** Système selon la revendication 9, dans lequel ladite unité de commande électronique (24) est en outre configurée, lors de la détermination de ladite position opérationnelle ajustée totale, pour calculer la moyenne desdites première et deuxième positions opérationnelles ajustées.

**11.** Système selon la revendication 9, dans lequel ladite unité de commande électronique (24) est en outre configurée, lors de la détermination de ladite position opérationnelle ajustée totale, pour pondérer chacune desdites première et deuxième positions opérationnelles ajustées en réponse aux distance réelles dudit premier capteur de position électrique ($44_1$) par rapport audit premier capteur de position magnétique ($46_1$) et audit deuxième capteur de position magnétique ($46_2$).

**12.** Système selon la revendication 1, dans lequel ladite unité de commande électronique (24) est en outre configurée pour afficher une image correspondant audit premier capteur de position électrique ($44_1$) sur un écran d'affichage en réponse à ladite première position opérationnelle ajustée.

13. Procédé pour faire naviguer un dispositif médical à l'intérieur d'un corps, comprenant les étapes suivantes:

déterminer une position opérationnelle pour un premier capteur de position électrique ($44_1$) sur ledit dispositif médical à l'intérieur d'un premier système de coordonnées, ledit premier système de coordonnées étant défini par un système de positionnement basé sur un champ électrique (18);

déterminer une position opérationnelle pour un premier capteur de position magnétique ($46_1$) sur ledit dispositif médical à l'intérieur d'un deuxième système de coordonnées, ledit deuxième système de coordonnées étant défini par un système de positionnement basé sur un champ magnétique, ledit premier capteur de position magnétique ($46_1$) étant disposé à proximité dudit premier capteur de position électrique ($44_1$);

appliquer une première fonction de mappage pour corréler lesdites positions opérationnelles dudit premier capteur de position électrique ($44_1$) et dudit premier capteur de position magnétique ($46_1$), ladite première fonction de mappage générant une position mappée pour ledit premier capteur de position magnétique ($46_1$) dans ledit premier système de coordonnées en réponse à ladite position opérationnelle dudit premier capteur de position magnétique ($46_1$) dans ledit deuxième système de coordonnées; et

déterminer une première position opérationnelle ajustée pour ledit premier capteur de position électrique ($44_1$) dans ledit premier système de coordonnées en réponse à ladite position mappée pour ledit premier capteur de position magnétique ($46_1$).

FIG.1

EP 2 854 634 B1

FIG.2

EP 2 854 634 B1

DETERMINE PLANNING POSITIONS FOR ELECTRICAL POSITION SENSOR 44 — 62

DETERMINE PLANNING POSITIONS FOR MAGNETIC POSITION SENSOR 46 — 64

MULTIPLE ELECTRICAL POSITION SENSORS?

NO

YES

AVERAGE PLANNING POSITIONS FOR MULTIPLE ELECTRICAL POSITION SENSORS 44 — 66

TOO MANY PLANNING POSITIONS?

NO

YES

COMPARE DISTANCE BETWEEN PLANNING POSITIONS — 68

DISTANCE TOO SMALL?

NO

YES

DISCARD ONE OF THE PLANNING POSITIONS — 70

FIG.3B

FIG. 3A

EP 2 854 634 B1

FIG.3A

COMPUTE MAPPING FUNCTION gFS — 72

DETERMINE VIRTUAL PLANNING POSITION FOR MAGNETIC POSITION SENSOR 46 — 76

DETERMINE VIRTUAL PLANNING POSITION FOR ELECTRICAL POSITION SENSOR 44 — 78

COMPUTE MAPPING FUNCTION lgFS — 74

DETERMINE OPERATING POSITION FOR ELECTRICAL POSITION SENSOR 44 — 80

DETERMINE OPERATION POSITION FOR MAGNETIC POSITION SENSOR 46 — 82

APPLY MAPPING FUNCTION lgFS TO OBTAIN MAPPED POSITION FOR SENSOR 46 IN COORDINATE SYSTEM 50 — 84

DETERMINE DIFFERENCE BETWEEN OPERATING POSITION OF SENSOR 44 AND MAPPED POSITION OF SENSOR 46 — 88

MODIFY OPERATING POSITION OF SENSOR 44 BY DIFFERENCE TO OBTAIN ADJUSTED OPERATING POSITION — 90

86

MORE MAGNETIC SENSORS 46?

YES

NO

FIG.3C

FIG.3B

FIG.3C

FIG.4

**EP 2 854 634 B1**